# EUROPÄISCHE PATENTSCHRIFT

(11) **EP 0 786 456 B1**
(45) Veröffentlichungstag und Bekanntmachung des Hinweises auf die Patenterteilung: **06.05.1999**
(21) Anmeldenummer: 97105185.9
(22) Anmeldetag: 02.09.1993
(51) Int. Cl.: C07D 249/10, C07D 257/04, C07D 471/04, C07D 487/04, C07C 47/19, C07C 45/75

(54) **Additionsverbindungen von Triazolium- oder Tetrazoliumsalzen**
Addition compounds of triazolium- or tetrazolium-salts
Composés d'addition de sels de triazolium ou de tétrazolium

(30) Priorität: 11.09.1992 DE 4230466
(43) Veröffentlichungstag der Anmeldung: 30.07.1997
(62) Teilanmeldung aus: 93114033.9
(73) Patentinhaber: BASF AKTIENGESELLSCHAFT, 67056 Ludwigshafen (DE)
(72) Erfinder: Teles, Joaquim Henrique, Dr., 67059 Ludwigshafen (DE); Melder, Johann-Peter, Dr., 67141 Neuhofen (DE); Gehrer, Eugen, Dr., 67069 Ludwigshafen (DE); Harder, Wolfgang, Dr., 69469 Weinheim (DE); Ebel, Klaus, Dr., 68623 Lampertheim (DE); Gröning, Carsten, Dr., 68259 Mannheim (DE); Meyer, Regina, Dr., 67136 Fussgönheim (DE)

(56) Entgegenhaltungen:
- JOURNAL FÜR PRAKTISCHE CHEMIE, CHEMIKER-ZEITUNG, Bd. 334, Nr. 3, 1992, HEIDELBERG DE, Seiten 231-6, XP002031049 H.G.O. BECKER ET AL.: "Kinetics and mechanism of ring cleavage of 1-benzyl-4-phenyl-1,2,4-triazolium chloride"

## Beschreibung

Die vorliegende Erfindung betrifft ein Verfahren zur katalytischen Herstellung von Kondensationsprodukten des Formaldehyds.

H.G.O. Becker et al (J. prakt. Chem. 334, 231 (1992)) untersuchten die Kinetik und den Mechanismus der Ringspaltung von 1-Benzyl-4-phenyl-1,2,4-triazolium-chlorid zum 4-Benzyl-1-formyl-1-phenyl-amidrazon mittels UV-spektrophotometrischer Verfolgung der Umsetzung des Triazoliumsalzes bei basischen pH-Werten in gepuffertem wäßrigem Methanol. Auf der Basis dieser Messungen, reaktionskinetischen Berechnungen und Überlegungen zu einem möglichen Reaktionsmechanismus postulierten Becker et al, daß diese Umsetzung über die Bildung der Verbindung 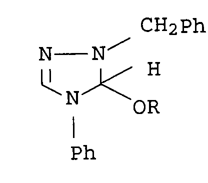 R = H, Methyl
als reaktiver Zwischenstufe verlaufen sollte. Ein spektroskopischer Nachweis dieser Verbindung, deren spektroskopische Charakterisierung und deren Isolierung gelang nicht.

Seit den Arbeiten von J. Castells (Tetrahedron Letters, 21∼ 4517 bis 4520, (1980)) ist bekannt, daß man Thiazoliumylide als Katalysatoren für die katalytische Umpolung von Formaldehyd und somit für dessen Selbstkondensation zu Dihydroxyaceton verwenden kann und ebenso für die entsprechende Umsetzung von höheren Aldehyden zu Acyloinen.

Mit Thiazoliumyliden als Katalysatoren erhält man bei der Acyloinkondensation von Formaldehyd hauptsächlich Ketone, insbesondere Dihydroxyaceton (DNA). Aufgabe der vorliegenden Erfindung war es, ein Verfahren sowie neue Katalysatoren für die Kondensation von Formaldehyd zu finden, die bei dieser Reaktion ein anderes Produktspektrum liefern als Thiazoliumylide und die insbesondere die Herstellung der Kondensationsprodukte Glykolaldehyd und/oder Glycerinaldehyd in guten Selektivitäten ermöglichen sollten. Insbesondere Glykolaldehyd kann nicht mit Hilfe der Thiazoliumylid-Katalysatoren erhalten werden. Glykolaldehyd und Glycerinaldehyd sind wertvolle Zwischenprodukte zur Herstellung von Ethylenglykol, Glyoxal, Glycerin sowie zur Synthese von Wirkstoffen.

Dementsprechend wurde ein Verfahren zur katalytischen Herstellung von Kondensationsprodukten des Formaldehyds gefunden, das dadurch gekennzeichnet ist, daß man Formaldehyd oder Formaldehyd-liefernde Verbindungen mit Katalysatoren umsetzt, die aus Triazoliumsalzen der Formel 1 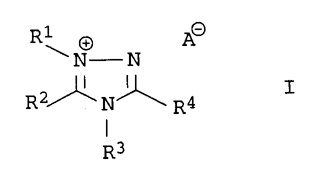 oder Tetrazoliumsalzen der Formel II 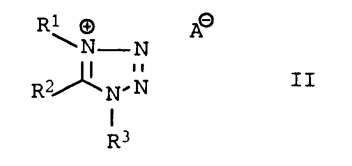 in denen
R¹ und R³ gleich oder verschieden sind und für aliphatische Gruppen mit 1 bis 30 Kohlenstoffatomen, für gegebenenfalls substituierte Arylgruppen, für gegebenenfalls substituierte Aralkylgruppen und/oder für gegebenenfalls substituierte Heteroarylgruppen stehen,
R² Wasserstoff, die Hydroxymethylengruppe -CH₂OH oder die Hydroxyhydroxymethylen-methylidin-gruppe -CH(OH)(CH₂OH) darstellt, und in der
R⁴ ein Halogenatom, eine aliphatische Gruppe mit 1 bis 30 Kohlenstoffatomen, eine gegebenenfalls substituierte Acylgruppe, eine gegebenenfalls substituierte Aralkylgruppe, eine gegebenenfalls substituierte Heteroarylgruppe, eine Alkoxygruppe -OR⁵, eine Thioethergruppe -SR⁶, eine Aminogruppe -NR⁷R⁸, eine Acylgruppe COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R³ gemeinsam mit dem Rest R⁴ eine C₃- bis C₅-Alkylen- oder Alkenylen- oder eine C₆- bis C₁₄-Arylen-, eine C₇- bis C₁₄-Aralkylen- oder C₈- bis C₁₄-Aralkenylenbrücke bildet, und in der
A das Äquivalent eines ein- oder mehrfach negativ geladenen Anions zur elektrischen Neutralisierung der Ladung des Triazoliumkations ist,
in Gegenwart einer Hilfsbase erzeugt worden sind.

Als Kondensationsprodukte des Formaldehyds werden im folgenden Glykolaldehyd, Glycerinaldehyd, Dihydroxyaceton und Tetrosen bezeichnet.

Bei den erfindungsgemäß zu verwendenden Katalysatoren handelt es sich somit um Katalysatoren, die aus 1,2,4-Triazoliumsalzen I oder Tetrazoliumsalzen II in Gegenwart einer Hilfsbase erzeugt werden. Da die Reste R¹, R³ und R⁴ in der Regel lediglich einen Einfluß auf das Löslichkeitsverhalten der Triazolium- oder Tetrazoliumsalze haben sowie deren Reaktivität und Selektivität bezüglich der Bildung der verschiedenen Formaldehydkondensationsprodukte modifizieren, können diese Reste eine Vielzahl von Bedeutungen haben.

So können R¹, R³ und R⁴ gleich oder verschieden sein und für aliphatische Gruppen mit 1 bis 30 Kohlenstoffatomen, wie C₁- bis C₃₀-Alkyl-, vorzugsweise C₁- bis C₁₀-Alkylgruppen, C₂- bis C₃₀₋, vorzugsweise C₂- bis C₁₀-Alkenyl- oder Alkinylgruppen mit 1 oder 2, vorzugsweise nur mit einer Mehrfachbindung, C₃- bis C₂₀-, vorzugsweise C₃- bis C₁₀-Cycloalkyl- oder -alkenyl-Gruppen, C₃- bis C₂₀-Heterocycloalkyl- oder -alkenyl-Gruppen, wie Piperidinyl-, Piperazinyl-, Pyrrolidinyl-, Imidazolidinyl-, Tetrahydrothienyl-, Tetrahydrofuranyl-, Tetrahydropyranyl-, Thiazolidinyl-, Oxazolidinyl-, Imidazolinyl-, Thiazolinyl-, Oxazolinyl- oder Kronenether-Gruppen, über ein Kohlenstoffatom an den Triazolium- oder Tetrazoliumring gebundene C₂- bis C₃₀-, vorzugsweise C₂- bis C₁₀-Alkoxygruppen mit einem oder mehreren, vorzugsweise nur mit einem Sauerstoffatom in der Etherkette, C₁- bis C₃₀-, vorzugsweise C₁- bis C₁₀-Fluor-, Chlor- und/oder Brom-, vorzugsweise Fluor- und/oder Chlor-, insbesondere Fluor-enthaltende Halogenalkylgruppen mit einem oder mehreren, vorzugsweise mit 1 bis 3 Halogenatomen, im Falle von Fluoralkylgruppen vorteilhaft auch perfluorierte Fluoralkylgruppen, über ein Kohlenstoffatom an den Triazolium- oder Tetrazoliumring gebundene Aminogruppen, wie C₂-bis C₃₀-, vorzugsweise C₂- bis C₁₀-sekundäre Aminogruppen, C₃- bis C₃₀-, vorzugsweise C₃- bis C₂₁-tertiäre Aminogruppen, für gegebenenfalls substituierte Arylgruppen, vorzugsweise C₆- bis C₁₄-Arylgruppen, insbesondere für Phenyl-, Naphthyl-, Anthryl- oder Phenanthryl-Gruppen, für gegebenenfalls substituierte C₇- bis C₂₀-Aralkylgruppen, insbesondere die Benzyl-, Phenylethylen- oder Naphthylmethylen-Gruppe, für gegebenenfalls substituierte C₂- bis C₁₅-Heteroarylgruppen mit 1 bis 3 Stickstoffatomen oder einem Sauerstoff-oder Schwefelatom oder mit 1 bis 2 Stickstoffatomen und einem Sauerstoff- oder Schwefelatom im Ring, wie die Furanyl-, Thienyl-, Pyrrolyl-, Pyrazolyl-, Imidazolyl-, Oxazolyl-, Thiazolyl-, Isothiazolyl-, Isoxazolyl-, Pyridinyl-, Pyrimidinyl-, Pyrazinyl-, Chinolinyl-, Naphthyridinyl-, 1,2,4-Triazolyl- oder Acridinyl-Gruppen stehen.

Sowohl die aliphatischen als auch die aromatischen und selbstverständlich auch die Aralkylreste können einfach oder mehrfach, vorzugsweise nicht mehr als dreifach, mit Halogenatomen, Nitro-, Hydroxy-, Cyano-, Alkyl-, Alkoxy- oder Aminogruppen substituiert sein. Da diese Substituenten in der Regel eine nur geringfügige Wirkung auf die katalytische Aktivität der aus den Verbindungen I, II, III bzw. IV erzeugten Katalysatoren ausüben, werden, hauptsächlich aufgrund der kostengünstigeren Herstellung, die obengenannten, unsubstituierten Reste R¹, R³ und R⁴ bevorzugt verwendet.

Außer den obengenannten Bedeutungen kann der Rest R⁴ im Unterschied zu den Resten R¹ und R³ ein Wasserstoff, eine Nitro- oder Cyanogruppe, ein Halogenatom, ausgewählt aus Fluor, Chlor oder Brom, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸, eine Acylgruppe -COR⁹ oder eine Estergruppe- COOR¹⁰ sein. Die Reste R⁵, R⁶, R⁷, R⁸ und R⁹ können die gleichen Bedeutungen haben, wie sie oben für den Rest R¹ angegeben sind. Ebenso wie die Reste R¹ können auch die Reste R⁵, R⁶, R⁷, R⁸, R⁹ und R¹⁰ die für die Reste R¹ genannten Substituenten tragen, vorzugsweise, aufgrund der kostengünstigeren Herstellung, werden in der Regel aber unsubstituierte Reste R⁵, R⁶, R⁷, R⁸, R⁹ und R¹⁰ gewählt. Bevorzugte Reste R⁵, R⁶, R⁷, R⁸, R⁹ und R¹⁰ sind C₁- bis C₃₀-, insbesondere C₁- bis C₁₀-Alkylgruppen, C₆- bis C₁₀-Arylgruppen, insbesondere die Phenyl- oder Naphthylgruppe sowie C₇-bis C₂₀-, vorzugsweise C₇- bis C₁₄-Aralkylgruppen, insbesondere die Benzyl-, Phenylethylen- oder Naphthylmethylen-Gruppe. Ein weiterer bevorzugter Rest R⁷ ist die Hydroxethylengruppe.

Der Rest R⁴ kann weiterhin gemeinsam mit dem Rest R³ eine C₃- bis C₅-Alkylen- oder Alkenylenbrücke- eine C₆- bis C₁₄-Arylenbrücke, vorzugsweise eine o-Phenylen-, o-Naphthylen-, 1,8-Naphthylen-, o-Fluorenylen-, 5,4-Fluorenylen-, o-Phenanthrylen-, 5,4-Phenanthrylen-, 9,10-Phenanthrylen-, o-Anthrylen-, 1,9-Anthrylen- oder eine 2,2'-Biphenylenbrücke, eine C₇- bis C₁₄-Aralkyl- oder Aralkenylenbrücke bilden, wobei diese verbrückenden Reste R³∩R⁴ einfach oder mehrfach, vorzugsweise nicht mehr als 3fach mit Halogenatomen, Nitro-, Hydroxy-, Cyano-, Alkyl-, Alkoxy- oder Aminogruppen substituiert sein können. Da diese Substituenten in der Regel eine nur geringfügige Wirkung auf die katalytische Aktivität der betreffenden aus I erzeugten Katalysatoren ausüben, werden in der Regel, hauptsächlich auf Grund ihrer kostengünstigeren Herstellung, unsubstituierte verbrückende Reste R³∩R⁴ bevorzugt. Da sowohl der Rest R³ als auch der Rest R⁴ die Heteroatome Stickstoff, Sauerstoff oder Schwefel enthalten können, kann auch der verbrückende Rest R³∩R⁴ diese Heteroatome enthalten, vorzugsweise enthält der verbrückende Rest R³∩R⁴ nicht mehr als 2, insbesondere nicht mehr als eines dieser Heteroatome.

Der Rest R², der sich am mutmaßlich katalytisch aktiven Zentrum der Triazoliumverbindungen I bzw. Tetrazoliumverbindungen II befindet, kann Wasserstoff, die Hydroxymethylengruppe -CH₂OH oder die Hydroxy-hydroxmethylen-methylidin-Gruppe -CH(OH)(CH₂OH) darstellen.

Die Anionen, die das Anion-Äquivalent A zur elektrischen Neutralisierung der Ladung des Triazolium- bzw. Tetrazolium-Kations bilden, können im Prinzip beliebig gewählt werden, vorzugsweise werden aber die nicht-nukleophilen Anionen von Mineralsäuren oder starken Carbonsäuren gewählt. Diese Anionen können ein- oder mehrfach, bevorzugt nicht mehr als dreifach negativ geladen sein. Derartige mehrfach negativ geladene Anionen können die ihrer negativen Ladung entsprechende Anzahl Triazolium- bzw. Tetrazolium-Kationen elektrisch neutralisieren und elektrostatisch, also salzartig, an sich binden. Das Anion-Äquivalent A entspricht somit der zur elektrischen Neutralisation einer molaren Menge des Triazolium- bzw. Tetrazolium-Kations benötigten molaren Menge eines einfach oder mehrfach negativ geladenen Anions dividiert durch dessen Ladungszahl.

Geeignete Anionen sind beispielsweise die Anionen der Halogenide, wie Fluorid, Chlorid, Bromid oder Jodid, Nitrat, Tetrafluoroborat, Tetraphenylborat, Hexafluorophosphat, Hexachloroplatinat, Perchlorat, Sulfat, Phosphat, Trifluoracetat, Methansulfonat oder Toluolsulfonat. Ebenso können saure Kationenaustauscher in ihrer anionischen Form, beispielsweise Polyacrylate, sulfonierte Phenol-Formaldehyd-Harze oder sulfoniertes Polystyrol als Polyanionen wirken. Vorzugsweise werden die Halogenide, Nitrat, Tetrafluoroborat sowie Perchlorat als Anion verwendet.

Die Wirkungsweise der erfindungsgemäß eingesetzten Triazoliumsalze I oder Tetrazoliumsalze II ist noch weitgehend unbekannt und es können lediglich Mutmaßungen über den dem erfindungsgemäßen Verfahren zugrunde liegenden chemischen Mechhanismus aufgestellt werden. Die Ergebnisse aller bisherigen Experimente deuten jedoch darauf hin, daß das Triazoliumsalz I bzw. des Tetrazoliumsalz II durch die Hilfsbase in 5-Position des Triazolium- bzw. Tetrazoliumringes deprotoniert wird, wobei das mit dem Carben IA bzw. IIA mesomere Ylid IB bzw. IIB gebildet wird (siehe Gleichung (1)), 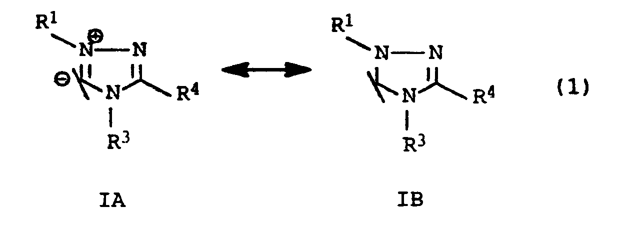 von dem vermutet wird, daß es die eigentliche katalytisch aktive Spezies ist. Es ist zwar möglich, daß unter den Reaktionsbedingungen auch noch andere katalytisch aktive Spezies dieser Triazoliumverbindungen vorliegen, die sich von den Yliden IA bzw. IIA/Carbenen IB bzw. IIB ableiten lassen und mit diesen im Gleichgewicht stehen, Voraussetzung für die Entwicklung einer katalytischen Aktivität, scheint jedoch das Durchlaufen der reaktiven Zwischenstufe des Triazolium- bzw. Tetrazolium-Ylids/Carbens zu sein, auf welche Weise und ausgehend von welchen Ausgangsmaterialien dieses auch immer generiert werden mag. Diese Darlegungen sind jedoch lediglich als Versuch einer Erklärung der erfindungsgemäßen Umsetzung zu betrachten. Sollte zukünftig einmal festgestellt werden, daß andere reaktive Zwischenstufen als die hier postulierten, die erfindungsgemäße Umsetzung katalysieren, so ist dies für den Schutzumfang der vorliegenden Anmeldung als unerheblich zu betrachten, da diese Spezies schließlich durch die erfindungsgemäßen Maßnahmen erzeugt werden.

Gestützt werden die obendargelegten Annahmen durch den Umstand, daß bei der Umsetzung der Triazoliumsalze I bzw. der Tetrazoliumsalze II mit Formaldehyd in Substanz isolierbare Triazoliumsalz- bzw. Tetrazoliumsalz-Formaldehyd-Additionsverbindungen entstehen, die den Triazoliumverbindungen I bzw. Tetrazoliumverbindungen II mit R² = Hydroxymethylen -CH₂OH oder Hydroxy-hydroxymethylen-methylidin -CH(OH)(CH₂OH) entsprechen und die, setzt man sie separat mit Formaldehyd in Gegenwart einer Hilfsbase um, ebenfalls katalytisch aktiv sind und die Kondensation von Formaldehyd, insbesondere zu Glykolaldehyd, Glycerinaldehyd und Tetrosen katalysieren.

Einen weiteren Hinweis darauf, daß Position 5 des Triazolium- bzw. Tetrazoliumringes höchstwahrscheinlich das katalytisch aktive Zentrum dieser Katalysatoren ist, gibt der Umstand, daß bei der Umsetzung von Alkoholaten, beispielsweise Methanolaten, oder Thiolaten mit den Triazoliumsalzen I bzw. Tetrazoliumsalzen II Alkoholat- bzw. Thiolat-Additionsprodukte der Formel III 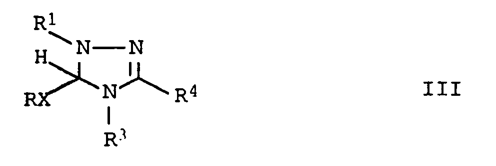 bzw. der Formel IV 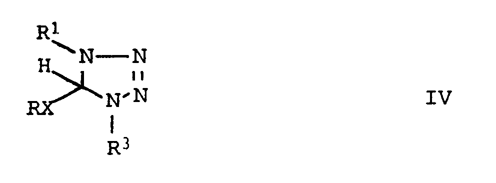 isoliert werden können, in der X für Sauerstoff oder Schwefel steht, R eine C₁- bis C₄-Alkylgruppe, vorzugsweise die Methylgruppe ist und in der R¹, R³ und R⁴ die obengenannte Bedeutung haben. Setzt man diese Additionsverbindungen III oder IV an Stelle der Triazoliumsalze I bzw. Tetrazoliumsalze II mit Formaldehyd unter den üblicherweise im erfindungsgemäßen Verfahren angewandten Reaktionsbedingungen, jedoch in Abwesenheit der Hilfsbase, um, so bilden sich ebenfalls Formaldehydkondensationsprodukte, insbesondere Glykolaldehyd, Glycerinaldehyd und Tetrosen. Wahrscheinlich wird aus den Verbindungen III bzw. IV unter den üblicherweise angewandten Reaktionsbedingungen der Alkohol bzw. das Thiol RXH wieder abgespalten und das mutmaßlich katalytisch aktive Ylid bzw. das Carben erzeugt, welches dann seine katalytische Aktivität entfalten kann.

Des weiteren ist es möglich, die Additionsverbindungen III bzw. IV allein, in fester Form oder in einem hochsiedenden Lösungsmittel zu erhitzen, dabei den betreffenden Alkohol bzw. das betreffende Thiol abzuspalten und auf diese Weise Verbindungen zu erzeugen, die besonders aktive Katalysatoren für die Kondensation des Formaldehyds sind. Höchstwahrscheinlich entsteht bei dieser Thermolyse das entsprechende Ylid bzw. das Carben oder eine diesen Spezies äquivalent wirkende Verbindung.

Somit ergeben sich für das erfindungsgemäße Verfahren zur Herstellung von Kondensationsprodukten des Formaldehyds dreierlei Ausgestaltungen:
α) Verwendung der Triazoliumsalzverbindungen I oder Tetrazoliumsalzverbindungen II als Katalysatoren in Gegenwart einer Hilfsbase.
β) Verwendung der Additionsverbindungen III bzw. IV als Katalysatoren.
γ) Verwendung der durch die thermische Eliminierung der Alkohole ROH bzw. Thiole RSH aus den Additionsverbindungen III bzw. IV erhaltenen Thermolyseprodukte als Katalysatoren.

Allen diesen drei Ausgestaltungen des erfindungsgemäßen Verfahrens ist gemäß den vorausgegangenen Darlegungen gemein, daß die Kondensation des Formaldehyds letztendlich von der gleichen katalytisch aktiven Spezies katalysiert wird. Alle diese drei Ausgestaltungen sind daher einander äquivalent, obgleich diese drei Verfahrensvarianten unterschiedliche Vorteile haben, die im Einzelfall den Ausschlag für die Anwendung der einen oder der anderen Variante geben:

Variante α) hat den Vorteil, daß die Triazoliumverbindungen I bzw. Tetrazoliumverbindungen II nicht weiter derivatisiert werden müssen. Die Varianten β) und γ) haben den Vorteil, daß sie in Abwesenheit einer Hilfsbase durchgeführt werden können. Da die Anwesenheit einer Hilfsbase im Reaktionsgemisch gegebenenfalls zu unerwünschten Isomerisierungsreaktionen und Disproportionierungsreaktionen der Formaldehydkondensationprodukte Anlaß geben kann, wiegt dieser Vorteil im Einzelfall besonders schwer. Die gemäß Variante γ) eingesetzten katalytisch aktiven Verbindungen sind besonders aktive Katalysatoren. Besonders geeignet zur Durchführung der Variante β) des erfindungsgemäßen Verfahrens sind die Methanolat-Additionsverbindungen IIIa 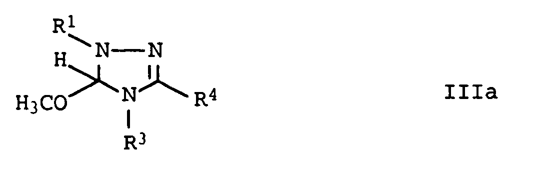 bzw. IVa.

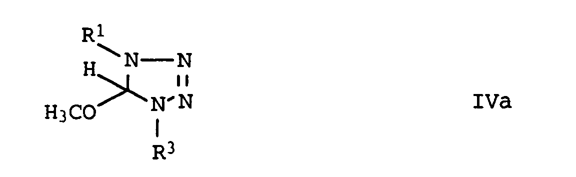

Das erfindungsgemäße Verfahren wird zweckmäßigerweise in Gegenwart eines Lösungsmittels durchgeführt. Als Lösungsmittel eignen sich grundsätzlich ein sehr breites Spektrum von Lösungsmitteln, wie Alkohole, z.B. Methanol, Ethanol, Propanol, Cyclohexanol, 2-Ethylhexanol und Hexadecylalkohol, Amide, z.B. Dimethylformamid (DMF), Dibutylformamid, Harnstoffe, wie Dimethylethylenharnstoff, Dimethylpropylenharnstoff, Carbonate, z.B. Propylencarbonat, Ethylencarbonat, aromatische Lösungsmittel, z.B. Toluol oder Xylol, Heterocyclen, z.B. Pyridin, N-Methylimidazol, N-Methylpyrrolidon, Ketone, z.B. Aceton, Ester, z.B. Essigsäureethylester, Ether, z.B. Methyl-tert.-Butylether, Diethylenglykoldimethylether, Dimethoxyethan, Tetrahydrofuran, Dioxan, Nitroverbindungen, z.B. Nitrobenzol, Nitrotoluol, Nitromethan, tertiäre Amine, z.B. Triethylamin, halogenierte Kohlenwasserstoffe wie Chloroform, Dichlormethan, Chlorbenzol oder Dichlorbenzol, Sulfoxide, wie Dimethylsulfoxid, Sulfone, wie Sulfolan, Trioxan und Nitrile, z.B. Acetonitril oder Propionitril.

Die Menge des eingesetzten Lösungsmittels ist im allgemeinen nicht kritisch und von der Art des verwendeten Lösungsmittels abhängig, weshalb zweckmäßigerweise in einem Vorversuch die optimal einzusetzende Lösungsmittelmenge für das betreffende Lösungsmittel ermittelt wird. Als Hilfsbasen zur Aktivierung der Triazoliumsalze I oder Tetzazoliumsalze II können eine Vielzahl von Basen eingesetzt werden, die auf Grund ihrer Basenstärke in der Lage sind, die Triazoliumsalze I oder Tetrazoliumsalze II in 5-Position oder die 5-Hydroxymethylen- oder 5-((Hydroxy)-(hydroxymethylen)-methylidin))-Derivate der Triazoliumsalze I oder Tetrazoliumsalze II zu deprotonieren. Vorzugsweise werden dabei nicht-nukleophile Basen verwendet, beispielsweise tertiäre Amine mit 3 bis 30 Kohlenstoffatomen oder tertiäre cyclische Amine, insbesondere auch cyclische Amidine.

Geeignete tertiäre Amine sind beispielsweise Trimethylamin, Triethylamin, Tripropylamin, Tributylamin, Ethyldiisopropylamin, Decyldiethylamin, Tridecylamin, Chinuclidin, Diazabicyclo-[2,2,2]-Octan, N-Methylpiperidin, N-Ethylpiperidin, N-Propylpiperidin, N-Butylpiperidin, N,N'-Dimethylpiperazin, N-Methylmorpholin, Dimethylbenzylamin, Dibenzylmethylamin, Benzyldioctylamin, Benzyldiethylamin, Cyclohexyldiethylamin, Dicylcohexyldiethylamin, Dicyclohexylmethylamin, Dicyclohexylmethylamin, Dicyclohexylethylamin usw.. Besonders bevorzugt wird Triethylamin verwendet. Von den cyclischen Amidinen werden 1,5-Diazabicyclo-[4,3,0]-non-5-en, 1,8-Diazabicyclo-[5,4,0]-undec-7-en und 1,5,7-Triazabicyclo-[4,4,0]-dec-7-en vorzugsweise eingesetzt.

Es können auch polymere tertiäre Amine als Hilfsbasen verwendet werden, beispielsweise vernetzte Styrol-Divinylbenzol-Harze oder Phenol-Formaldehyd-Harze, die Seitenketten mit tertiären Aminogruppen tragen oder deren Arylgruppen mit Dialkylaminogruppen substituiert sind. Solche polymeren Amine werden üblicherweise als Anionentauscher eingesetzt.

Außerdem können auch aromatische Stickstoffbasen, wie Chinolin, Pyridin oder N-Alkylimidazole, insbesondere N-C₁- bis C₄-Alkylimidazole zur Deprotonierung der Triazoliumsalze I oder Tetrazoliumsalze II verwendet werden. Des weiteren können anorganische Basen, wie Alkalimetall- und Erdalkalimetall-Hydrogencarbonate, wie Alkalimetall- und Erdalkalimetallcarbonate oder Alkalimetallcarboxylate, insbesondere die Natrium- und Kaliumsalze der C₁- bis C₄-Carbonsäuren, benutzt werden.

Mit Hilfe der Verwendung polymerer tertiäre Amine in Form von Anionenaustauschern ist es möglich, aus den Triazoliumsalzen I oder Tetrazoliumsalze II Lösungen der entsprechenden Ylide/Carbene herzustellen, die keine Hilfsbase enthalten. Dazu ist es lediglich erforderlich, die Triazoliumsalze I oder Tetrazoliumsalze II an einem tertiäre Aminogruppen tragenden Anionenaustauscherharz zu deprotonieren, beispielsweise indem man eine Lösung des Triazoliumsalzes I oder Tetrazoliumsalzes II über ein solches Aniononaustauscherharz leitet, und den Formaldehyd bzw. die Formaldehyd-liefernde Verbindung der Lösung des so erzeugten Ylids/Carbens erst zusetzt, nachdem diese den Anionenaustauscher passiert hat. Diese Arbeitsweise hat den Vorteil, daß die Bildung von Nebenprodukten infolge der Cannizzaro-Reaktion der gebildeten Kondensationsprodukte weitgehend unterdrückt wird.

Im erfindungsgemäßen Verfahren kann der Formaldehyd in Form von Formaldehyd oder in Form von Formaldehyd-liefernden Verbindungen, z.B. als Paraformaldehyd, Formaldehydhalbacetallösungen, gasförmiger Formaldehyd oder als wäßrige Lösung (Formalin®) eingesetzt werden. Er wird im allgemeinen bei Temperaturen im Bereich von 20 bis 160°C, bevorzugt bei 50 bis 120°C und besonders bevorzugt bei 60 bis 80°C, zusammen mit dem organischen Lösungsmittel und dem Katalysator zweckmäßigerweise 5 bis 500 min., bevorzugt 15 bis 60 min. lang gerührt. Wird der Katalysator gemäß Verfahrensvariante β) aus den Verbindungen der Formel III oder IV in situ im Reaktionsgemisch erzeugt, werden im allgemeinen Temperaturen von 50 bis 160°C, vorzugsweise von 60 bis 100°C angewandt. Gelangen hingegen die nach Verfahrensvariante γ) in einer separaten Thermolyse erzeugten, katalytisch aktiven Verbindungen zum Einsatz, kann bei Temperaturen von 0 bis 160°C, vorzugsweise von 20 bis 120°C, gearbeitet werden.

Der angewandte Druck ist im allgemeinen für das erfindungsgemäße Verfahren nicht kritisch, es wird daher zweckmäßigerweise bei Atmosphärendruck oder unter dem Eigendruck des Reaktionssystems gearbeitet.

Das Molverhältnis von Formaldehyd zu Katalysator kann bei sämtlichen Ausgestaltungen des erfindungsgemäßen Verfahrens in einem Bereich von 10:1 bis 30 000:1 liegen. Bei Formaldehyd/Katalysator-Molverhältnissen von mehr als 200 kann vorteilhaft eine Base oder ein Puffer zum Abfangen der Säuren zugesetzt werden.

Wie bereits erwähnt, wird für die Durchführung des erfindungsgemäßen Verfahrens nach Verfahrensvariante β) oder γ) grundsätzlich keine Hilfsbase benötigt, da die Katalysatoren entweder in situ (Variante β)) oder ex situ (Variante γ)) durch Eliminierung von RXH, vorzugsweise durch die Eliminierung von Methanol, aus den Vorläuferverbindungen III bzw. IV erzeugt werden. Da jedoch während der Umsetzung als Folge von Nebenreaktionen in geringem Ausmaß Säuren entstehen können, beispielsweise Ameisensäure durch die Cannizzaro-Reaktion des Formaldehyds und diese Säuren den Katalysator desaktivieren können, kann der Zusatz geringer Mengen an Base, beispielsweise von solchen Basen wie sie zuvor für die Verwendung als Hilfsbasen geeignet genannt wurden, vorteilhaft sein, um diese Säuren abzupuffern oder zu neutralisieren. Dies kann insbesondere dann günstig sein, wenn man mit sehr kleinen Katalysatormengen arbeitet. Es können somit auch in diesen Fällen, die Mengen an Base verwendet werden, wie sie zuvor für Variante α) genannt wurden. Vorteilhaft werden aber geringere Basenmengen eingesetzt. Ist der eingesetzte Formaldehyd oder die Formaldehydliefernde Verbindung mit Säuren, insbesondere Ameisensäure, verunreinigt, so werden diese aus den obengenannten Gründen zweckmäßigerweise vor dem Einsatz im erfindungsgemäßen Verfahren neutralisiert oder die Wirkung dieser Säuren im erfindungsgemäßen Verfahren in situ durch Zusatz einer äquivalenten Basenmenge kompensiert.

Man kann die Reaktion sowohl in homogener Phase, als auch in Suspension oder auch in einem flüssigen Zweiphasensystem durchführen. Im letztgenannten Fall wird z.B. als Rohstoff wäßriger Formaldehyd eingesetzt und der Katalysator in einem organischen, mit Wasser nicht mischbaren Lösungsmittel, vorzugsweise in einem langkettigen, insbesondere in einem C₆- bis C₂₀-Alkohol, gelöst. Unter diesen Phasentransferbedingungen wird dabei der Formaldehyd in die organische Phase extrahiert, dort am Katalysator umgesetzt und das hydrophile Produkt (Glykolaldehyd, Glycerinaldehyd) in die wäßrige Phase zurückextrahiert.

Die im erfindungsgemäßen Verfahren verwendeten Triazoliumsalze I können nach bekannten Verfahren, beispielsweise durch die Alkylierung mesoionischer Verbindungen, beispielsweise die N-Alkylierung von Nitron mittels Alkylhalogeniden oder Dialkylsulfaten (vgl. Busch et al.: Ber 38, 4049 (1905)), durch die oxidative Entschwefelung der entsprechend substituierten Triazolin-5-thione (vgl. Z. Naturforsch. B, 25, 1421 (1970); J. Prakt. Chem., 330, 325 (1988)), welche wiederum durch die Lewis-Säure katalysierte Cyclisierung der entsprechenden Thiosemicarbazone, die aus den entsprechenden Isothiocyanaten nach deren Umsetzung mit einem Alkyl- oder Arylhydrazin zum betreffenden Thioharnstoff und dessen Alkylierung mit einem Aldehyd hergestellt werden können, erhältlich sind (Ber. 42, 4596 (1909); Ber. 34, 320 (1901)). Triazoliumsalze I, in denen R⁴ eine über das Heteroatom an den Triazoliumring gebundene Ether- oder Thioethergruppe ist, werden ebenfalls ausgehend von den entsprechenden Isocyanaten bzw. Isothiocyanaten durch deren Umsetzung mit einem Hydrazin zum entsprechenden Harnstoff- bzw. Thioharnstoffderivat, dessen Formylierung und Cyclisierung mit Ameisensäure und die anschließende Alkylierung des dabei erhaltenen 1,2,4-Triazolin-5-ons bzw. 1,2,4-Triazolin-5-thions mit einem Alkylierungsmittel synthetisiert (vgl. Ber. 42, 4596 (1909); J. Prakt. Chem. 67, 246 (1903); J. Prakt. Chem. 67, 263 (1903).

Polycyclische Triazoliumsalze I können aus sekundären Methylaminen nach deren N-Nitrosierung (Org. Synth., Coll. Vol. 2, 460 (1943)) und O-Alkylierung zum entsprechenden Alkoxi-diazeniumsalz in einer 1,3-dipolaren Cycloaddition mit den betreffenden N-Heterocyclen (s. Chem. Ber. 102, 3159 (1969)) hergestellt werden.

Triazoliumsalze I, die in 3-Position ein Wasserstoffatom tragen, können z.B. nach dem Verfahren von US-A-3 488 761 durch die Umsetzung von 1,2,4-Triazol mit Alkylierungs- oder Arylierungsmitteln, wie Alkylhalogeniden oder Dialkylsulfaten oder Arylhalogeniden, insbesondere Arylfluoriden, erhalten werden. Das 1,2,4-Triazol ist nach dem Verfahren von Ainsworth et al. J. Am. Chem. Soc. 77, 621 (1955) erhältlich. Alternativ können 3(H)-Triazoliumsalze I nach dem Verfahren von Boyd et al. J. Chem. Soc (C) 409 (1971) durch die Umsetzung der entsprechenden Oxadiazoliumsalze mit einem primären Amin erhalten werden.

Zur Erzeugung der Additionsverbindungen III oder IV setzt man im allgemeinen das betreffende Triazoliumsalz I oder Tetrazoliumsalz II in einem Lösungsmittel, beispielsweise einem Ether wie Tetrahydrofuran, Dioxan oder Dimethoxyethan oder einem Alkanol mit dem betreffenden Alkanolat- bzw. Thiolat RXMe um, in dem Me das Grammäquivalent eines Metallkations, vorzugsweise eines Alkalimetallkations, ist und X und R die zuvor genannte Bedeutung haben. Vorzugsweise werden die Alkanolate in dem Alkohol gelöst eingesetzt, aus dem sie erzeugt worden sind. Bei der Umsetzung der Alkanolate oder Thiolate RXMe mit dem Triazoliumsalz I oder Tetrazoliumsalz II arbeitet man im allgemeinen bei Temperaturen von 0 bis 100°C, vorzugsweise von 20 bis 50°C und wendet ein Molverhältnis Alkoholat bzw. Thiolat/Triazoliumsalz I bzw. Tetrazoliumsalz II von im allgemeinen 0,8 bis 1,5, vorzugsweise von 1 bis 1,2 an. Die Additionsprodukte III und IV können aus der so bereiteten Reaktionsmischung nach Entfernung des verwendeten Lösungsmittels und Abtrennung der entstandenen, in organischen Lösungsmitteln schwer löslichen Metallsalze, z.B. durch Filtration, isoliert werden und als solches erfindungsgemäß verwendet werden.

Zum Zwecke der Herstellung des Ylid/Carbens IA bzw. IIA/IB bzw. IIB werden die Additionsverbindungen III oder IV in Substanz oder in einem inerten, hochsiedenden Lösungsmittel z.B. einem Paraffin oder einem Sulfon, wie Sulfolan (Tetrahydrothiophen-1,1-dioxid), auf Temperaturen von 50 bis 160°C, vorzugsweise von 60 bis 140°C und besonders bevorzugt von 70 bis 120°C solange erhitzt, bis sämtliches Alkanol oder Thiol RXH aus III oder IV abgespalten ist. Dabei kann unter dem Eigendruck des Reaktionssystems gearbeitet werden, vorzugsweise arbeitet man jedoch bei vermindertem Druck. Das bei dieser Thermolyse erhaltene Thermolyseprodukt kann direkt als Katalysator im erfindungsgemäßen Verfahren eingesetzt werden.

### Beispiele

### Herstellung der Katalysatoren

Die Trlazoliumsalze Ia, Ib, Ic und Id, gemäß Tabelle wurden nach Eicher et al (Chem. Ber. 102, 3159 (1969)) hergestellt.

Das Triazoliumsalz Ie wurde, ausgehend von N,N'-Diphenyl-N-aminothioharnstoff (hergestellt nach: Ber. 42, 4596 (1909)) hergestellt:

Zu 20 g (82 mmol) N,N'-Diphenyl-N-aminothioharnstoff wurden langsam und unter Kühlung von außen 3,6 g (82 mmol) frisch destillierter Acetaldehyd gegeben. Dabei wurde die Reaktionsmischung erst zähflüssig, dann fest.

Das so erhaltene Produkt wurde nicht weiter gereinigt, sondern in 400 ml Ethanol aufgenommen und bei 60°C mit einer Lösung von 19,7 g (96 mmol) Eisen(III)chlorid in 50 ml Ethanol versetzt. Nach beendeter Zugabe wurde noch 1 h bei 60°C gerührt und anschließend das Ethanol bei vermindertem Druck abdestilliert. Der Rückstand wurde in Diethylether gelöst, und die etherische Phase mehrfach mit Wasser gewaschen und über Natriumsulfat getrocknet. Das Lösungsmittel wurde abdestilliert, und der Rückstand wurde aus Petroleumbenzin umkristallisiert.
1,4-Diphenyl-3-methyl-1,2,4(5H)-triazolin-5-thion:
Massenspektrum: Molpeak 267 m/e
Schmelzpunkt: 128 bis 130°C
Korrekte ¹H- und ¹³C-NMR-Spektren.

4,0 g (15 mmol) 1,4-Diphenyl-3-methyl-1,2,4(5H)-triazolin-5-thion wurden mit 60 ml konzentrierter Salpetersäure, 60 ml Wasser und 14 ml konzentrierter Perchlorsäure bei Raumtemperatur gerührt. Nach wenigen Minuten Reaktionsdauer begannn ein Feststoff auszufallen. Nach beendeter Reaktion wurde der Feststoff abfiltriert, nacheinander mit Wasser, Ethanol und Diethylether gewaschen und bei vermindertem Druck getrocknet.
1,4-Diphenyl-3-methyl-1,2,4-triazolium-perchlorat Ie:
Schmelzpunkt: 218°C (Zersetzung)
Korrekte ¹H- und ¹³C-NMR-Spektren.

Das Triazoliumsalz If, gemäß Tabelle, wurde nach den in der Beschreibung angegebenen Literaturverfahren über den Weg des 2,3,4-Triphenyl-substituierten 1,2,4-Triazolin-5-thion-derivats hergestellt.

### 1,3,4-Triphenyl-5-hydroxymethylen-1,2,4-triazolium-perchlorat Ig:

5,0 g (12,5 mmol) If, 4,0 g (133 mmol) Paraformaldehyd und 130 ml Tetrahydrofuran wurden 1 h im Glasautoklaven auf 80°C erhitzt. Nach dem Abkühlen wurde das Lösungsmittel bei vermindertem Druck abdestilliert und der halbfeste Rückstand mit 10 gew.-%iger Perchlorsäure gerührt. Der fest gewordene Rückstand wurde mit Wasser neutral gewaschen und aus Ethanol umkristallisiert
Schmelzpunkt: 182 bis 183°C,
Korrekte ¹H- und ¹³C-NMR-Sprektren

### 2,4-Diphenyl-5-(N-methyl-N-phenylamino)-1,2,4-triazoliumjodid Ih:

Nitron wurde nach Ber. 38, 4049 (1905) hergestellt und nach dem dort angegebenen Verfahren mit Methyljodid zu Ih umgesetzt.

2,0 g Ih (4,4 mmol) wurden in 40,0 g Tetrahydrofuran bei 55°C in einer Argonatmosphäre mit 5,3 g (176 mmol) Paraformaldehyd gelöst und 5 h unter Rückfluß gekocht. Der sich hierbei abscheidende Feststoff wurde in der Wärme abfiltriert. Die organische Lösung wurde mit 100 ml Dichlormethan verdünnt, mehrmals mit Wasser gewaschen und über Magnesiumsulfat getrocknet. Nach Abtrennung des Lösungsmittels bei vermindertem Druck wurde 1,4-Diphenyl-5-hydroxymethylen-3-(N-methyl-N-phenylamino)-1,2,4-triazolium-jodid Ii erhalten.
Schmelzpunkt: 200°C;
Korrekte ¹H- und ¹³C-NMR-Spektren

### 1,4-Diphenyl-3-(N-dodecyl-N-phenylamino)-1,2,4-triazolium-jodid Ij

2,0 g (6,4 mmol) Nitron und 9,5 g (32 mmol) Dodecyljodid wurden in 10 ml Toluol 24 h unter Argon auf 100°C erhitzt. Aus der abgekühlten Lösung wurde das ausgefallene, kristalline Produkt abfiltriert und aus Ethanol/Wasser umkristallisiert.
Schmelzpunkt: 129 bis 130°C;
Korrekte ¹H- und ¹³C-NMR-Spektren

### 1,4-Diphenyl-3-methoxy-1,2,4-triazolium-tetrafluoroborat Ik

2,0 g (8,4 mmol) 1,4-Diphenyl-3-hydroxy-1,2,4-triazoliumhydroxid-inneres Salz (hergestellt nach: J. Prakt. Chem. 67, 263 (1903)) wurden in 50 ml Dichlormethan suspendiert. Zu dieser Suspension wurden bei 0°C 1,3 g (9,2 mmol) festes Trimethyloxoniumtetrafluoroborat langsam gegeben. Die Reaktionsmischung wurde noch 4 h bei 0°C gerührt. Anschließend wurde der Feststoff abfiltriert, mit Dichlormethan gewaschen und aus Ethanol umkristallisiert. Man erhielt Ik in Form eines Gemisches, das zu 58 Gew.-% aus Ik, zu 23 Gew.-% aus der Ausgangsverbindung und zu 19 % aus dem Isomeren 1,4-Diphenyl-2-methyl-1,2,4-(3H)-triazolinium-3-on bestand.

Verbindung Il wurde nach dem Verfahren von J. Prakt Chem. 67, 246 (1903) hergestellt.

### 1,3,4-Triphenyl-5-H-5-methoxy-1,2,4-triazolin IIIα

Zu einer Lösung von 7,0 g (25 mmol) If in 150 ml Methanol wurden bei Raumtemperatur 1,4 g (26 mmol) Natriummethanolat, gelöst in 30 ml Methanol, gegeben. Das Methanol wurde anschließend unter vermindertem Druck abgezogen und der Rückstand in Diethylether aufgenommen. Das ungelöst bleibende Natriumperchlorat wurde abfiltriert. Nach Entfernung des Lösungsmittels wurde der zurückbleibende Feststoff aus Methanol umkristallisiert.
Schmelzpunkt: 136 bis 137°C;
Korrekte ¹H- und ¹³C-NMR-Spektren

### Ylid/Carben-Bildung aus IIIα

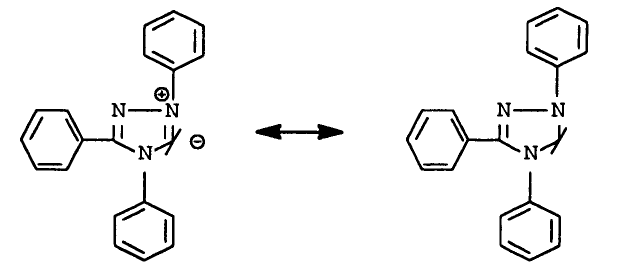

1,0 g (3 mmol) IIIα wurden solange bei 80°C und bei vermindertem Druck erhitzt, bis der für die Methanolabspaltung berechnete Gewichtsverlust eingetreten war. Dies war nach 16 h der Fall.

### Standardtests zur Bestimmung der Katalysatoraktivität

Die zuvor beschriebenen Triazoliumverbindungen Ia bis Ie wurden, wie folgt, auf katalytische Aktivität untersucht:

2,0 g (66 mmol) Paraformaldehyd wurden in 18 g DMF suspendiert. Zu dieser Suspension wurden 0,33 mmol der betreffenden Triazoliumverbindung gegeben (Formaldehyd/Katalysator-Molverhältnis = 200/1) und dieser Ansatz 30 min oder 1 h bei 80°C gerührt. Die bei den jeweiligen Tests verwendeten Hilfsbasen wurden jeweils in bezüglich des Triazoliumsalzes äquimolaren Mengen eingesetzt.

Die Reaktionsprodukte wurden folgendermaßen analysiert:
0,5 g Reaktionsprodukt wurden mit 10 g Oximierungsreagenz (5,0 g Butandiol-1,4 als innerer Standard für die Gaschromatographie und 70 g Hydroxylaminhydrochlorid in 1000 g Pyridin), vermischt und 1 h auf 70°C erwärmt. 1 ml dieser Lösung wurde dann mit 1 ml Hexamethyldisilazan und 1 ml Trimethylsilylchlorid vermischt und 0,5 bis 1 h bei Raumtemperatur stehengelassen bis Pyridiniumchlorid in großen Flocken ausfiel. Die Mischung wurde filtriert und die erhaltene Lösung direkt für die gaschromatographische Bestimmung der Reaktionsprodukte verwendet.

Die mit den einzelnen Katalysatoren erzielten Ergebnisse sind in der nachfolgenden Tabelle aufgelistet. In dieser Tabelle werden die Abkürzungen mit den folgenden Bedeutungen verwendet:
GA - Glykolaldehyd; GlyAld - Glycerinaldehyd;
DHA - Dihydroxyaceton; C₄ - Mischung aus C₄-Kohlenhydraten (Tetrosen); Et₃N - Triethylamin; C₅H₅H - Pyridin; NaOOCH - Natriumformiat; NaOAc - Natriumacetat

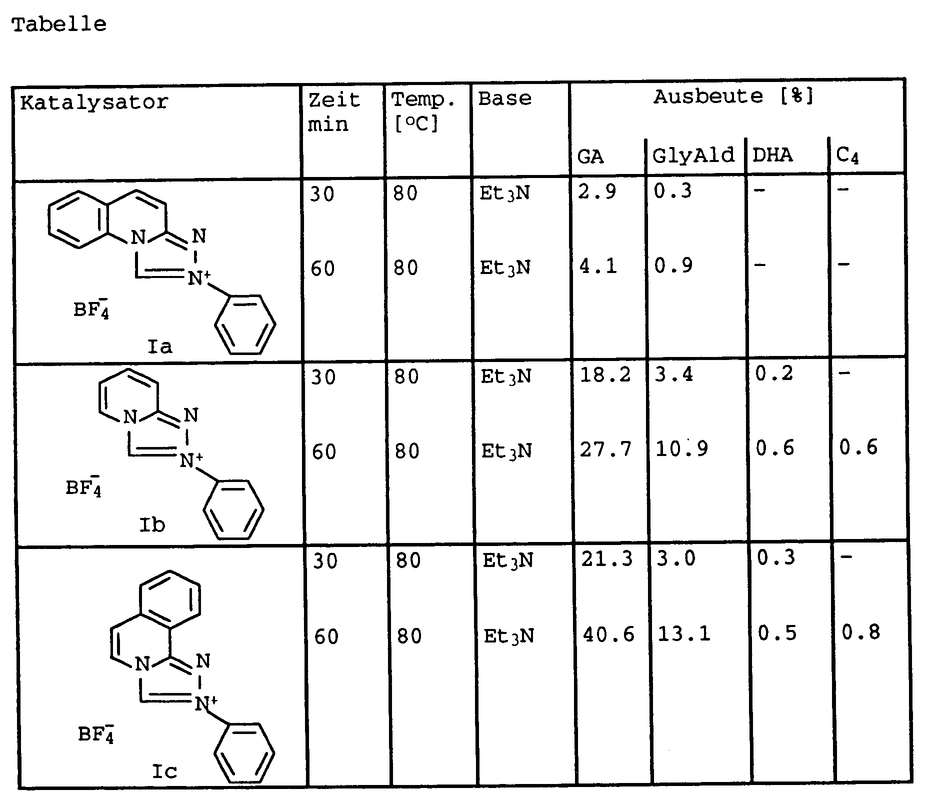

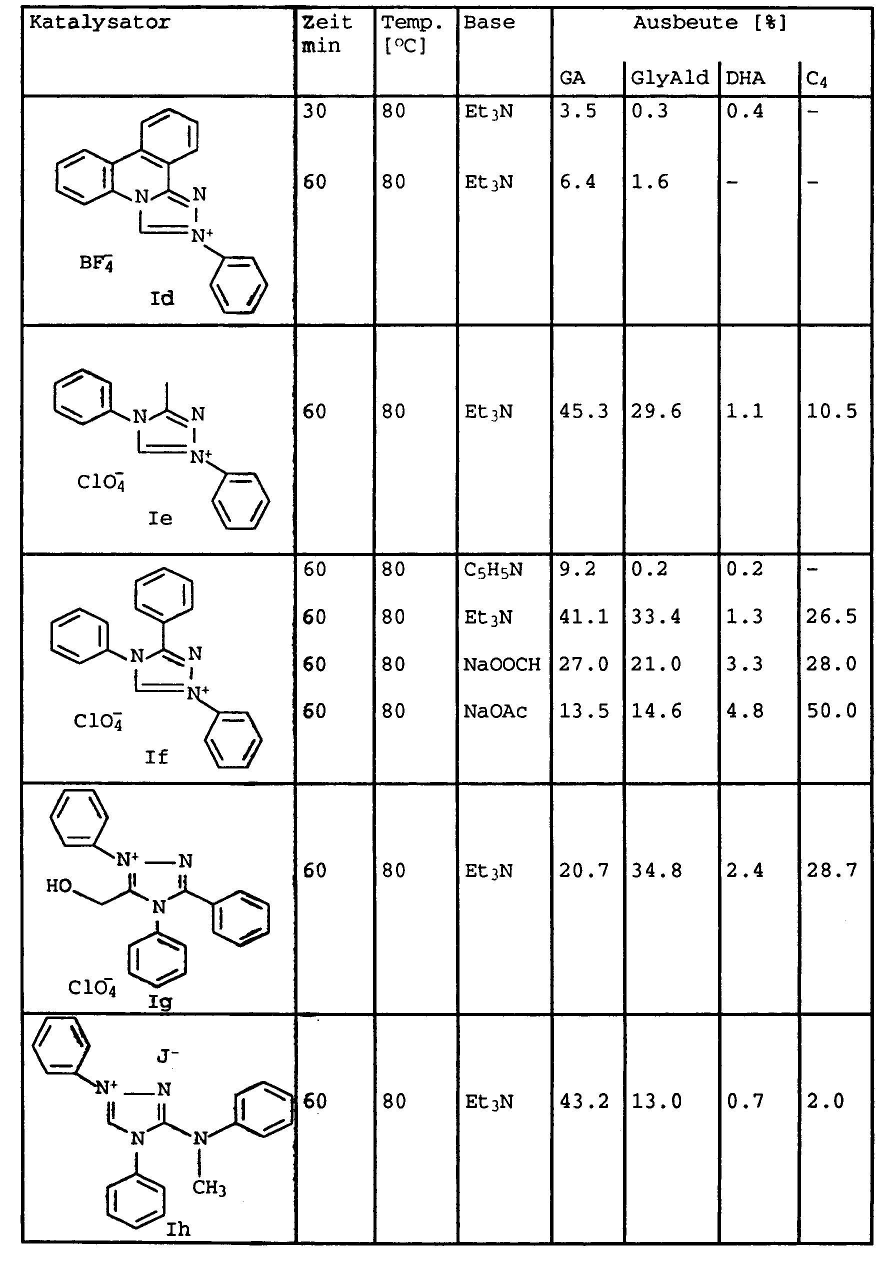

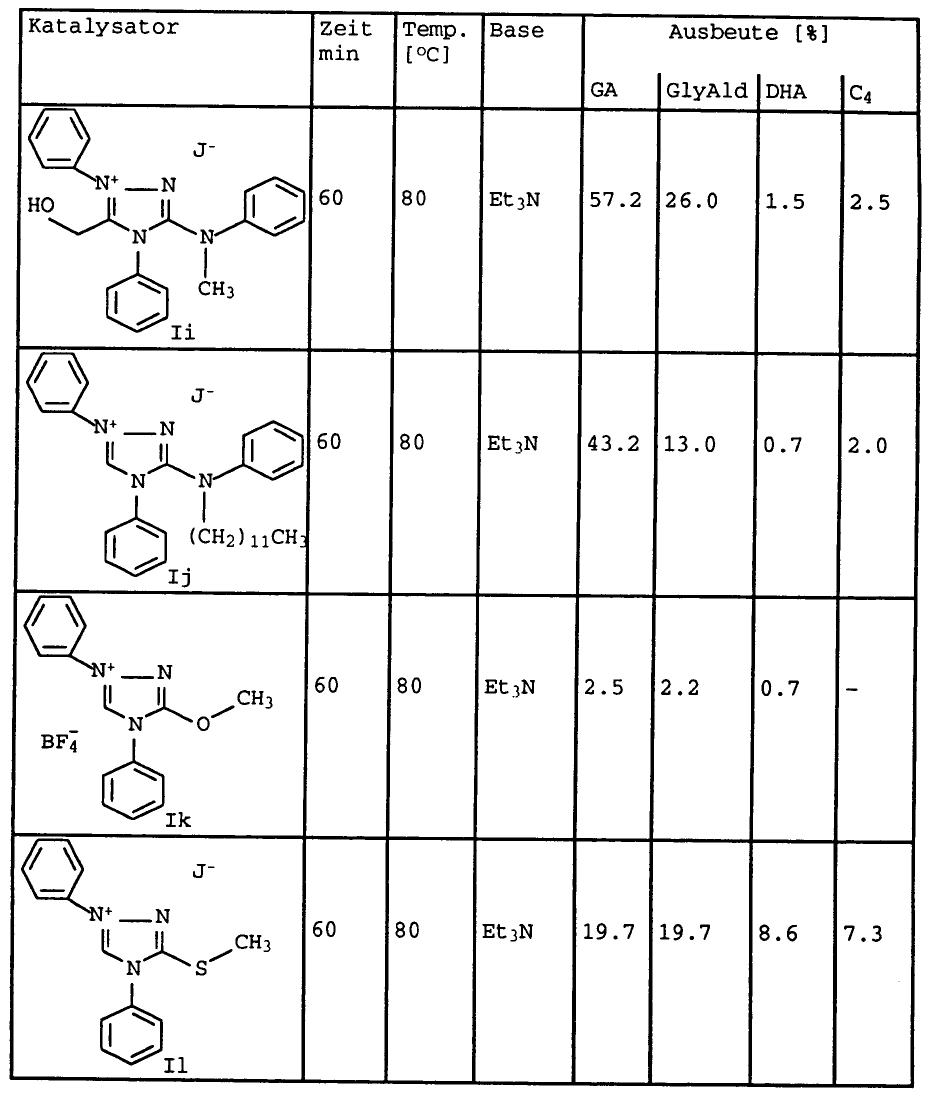

### Test von Verbindung IIIα auf Katalysatoraktivität

15 mg (0,066 mmol) IIIα wurden in 18 g Tetrahydrofuran mit Paraformaldehyd im Molverhältnis IIIα/Formaldehyd von 1/1000 bei 80°C 15 min gerührt.

Die entstandenen Reaktionsprodukte wurden nach dem zuvor beschriebenen Analysenverfahren gaschromatographisch analysiert.

### Ausbeute:

62 % Glykolaldehyd
1 % Glycerinaldehyd
11 % Dihydroxyaceton
1 % Tetrosen

Test des aus Verbindung IIIα durch thermische Eliminierung von Methanol erhaltenen Ylids/Carbens auf Katalysatoraktivität

15 mg des aus IIIα erhaltenen Ylids/Carbens wurden in 18 g Propionitril bei einer Temperatur von 80°C mit Paraformaldehyd im Molverhältnis Katalysator/Formaldehyd von 1/1500 45 min gerührt.

Die entstandenen Reaktionsprodukte wurden nach dem zuvor beschriebenen Analysenverfahren gaschromatographisch analysiert.

### Ausbeute:

60 % Glykolaldehyd
8 % Glycerinaldehyd
3 % Dihydroxyaceton
keine Tetrosen

### 1-p-Nitrophenyl-4-methyl-1,2,4-triazolium-jodid Im

5,0 g (2,6 mmol) 2-p-Nitrophenyl-1,2,4-triazol, 4,1 g (29 mmol) Methyljodid und 20 ml DMF wurden 24 h bei 100°C im Glasautoklaven gerührt. Nach Entfernung des Lösungsmittels wurde der Rückstand in 100 ml Dichlormethan aufgenommen, 30 min gerührt und der ausgefallene Feststoff abfiltriert.
Schmelzpunkt: 213 bis 216°C
Korrekte ¹H- und ¹³-C-NMR-Spektren

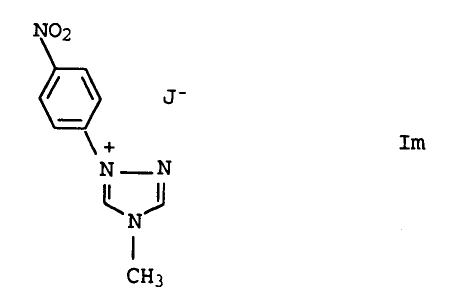

2 g (66 mmol) Paraformaldehyd wurden in 19 g Tetrahydrofuran suspendiert. Zu dieser Suspension wurden 0,33 mmol Im zugegeben und dieser Ansatz 120 min bei 80°C gerührt. Die Hilfsbase Triethylamin wurde in zu Im äquimolarer Menge verwendet. Die Analyse der Produkte erfolgte wie oben angegeben.

### Ausbeute:

46,2 % Glykolaldehyd
0,7 % Dihydroxyaceton
4,1 % Glycerinaldehyd.

## Patentansprüche

1. Additionsverbindungen von Triazoliumsalzen oder Tetrazoliumsalzen der Formel III 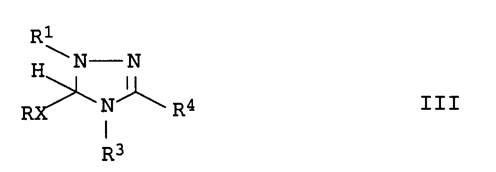 oder der Formel IV 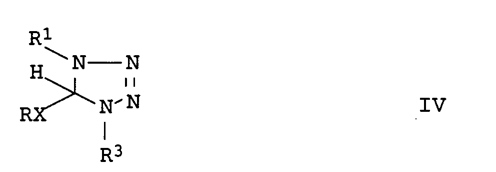
in denen X für Sauerstoff oder Schwefel steht und R eine C₁- bis C₄-Alkylgruppe ist.
R¹ und R³ gleich oder verschieden sind und für aliphatische Gruppen mit 1 bis 30 Kohlenstoffatomen, für gegebenenfalls substituierte Arylgruppen, für gegebenenfalls substituierte Aralkylgruppen und/oder für gegebenenfalls substituierte Heteroarylgruppen stehen, und
R⁴ Wasserstoff, ein Halogenatom, eine Nitro- oder Cyanogruppe, eine aliphatische Gruppe mit 1 bis 30 Kohlenstoffatomen, eine gegebenenfalls substituierte Arylgruppe, eine gegebenenfalls substituierte Aralkylgruppe, eine gegebenenfalls substituierte Heteroarylgruppe, eine Alkoxygruppe -OR⁵, eine Thioethergruppe -SR⁶, eine Aminogruppe -NR⁷R⁸, eine Acylgruppe - COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R³ gemeinsam mit dem Rest R⁴ eine C₃- bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet.

2. Additionsverbindungen von Triazoliumsalzen oder Tetrazoliumsalzen gemäß Anspruch 1, in denen
R¹ und R³ gleich oder verschieden sind und für C₁- bis C₃₀-Alkylgruppen, C₂- bis C₃₀-Alkenyl- oder Alkinylgruppen mit 1 oder 2 Mehrfachbindungen, C₃- bis C₂₀-Cycloalkyl- oder alkenylgruppen, C₃- bis C₂₀-Heterocycloakyl- oder -alkenylgruppen, über ein Kohlenstoffatom an den Triazoliumring gebundene C₂- bis C₃₀-Alkoxygruppen mit einem oder mehreren Sauerstoffatomen in der Etherkette, C₁- bis C₃₀-Fluor, Chlor- und/oder Brom- enthaltende Halogenalkylgruppen mit einem oder mehreren Halogenatomen, über ein Kohlenstoffatom an den Triazoliumring gebundene C₂- bis C₃₀-sekundäre Aminogruppen oder C₃- bis C₃₀-tertiäre Aminogruppen, für gegebenenfalls substituierte C₆- bis C₁₄-Arylgruppen, für gegebenenfalls substituierte C₆-bis C₁₄-Aralkylgruppen, für gegebenenfalls substituierte C₇-bis C₂₀-Aralkylgruppen, für gegebenenfalls substituierte C₂-bis C₁₅-Heteroarylgruppen mit 1 bis 3 Stickstoffatomen oder einem Sauerstoff- oder Schwefelatom oder mit 1 bis 2 Stickstoffatomen und einem Sauerstoff- oder Schwefelatom im Ring,
und der Rest R⁴ den Resten R¹ oder R³ gleich oder verschieden ist oder ein Wasserstoffatom, eine Nitro- oder Cyanogruppe, ein Fluor-, Chlor- oder Bromatom, eine über das Sauerstoffatom an den Triazoliumring gebundene Alkoxygruppe -OR⁵, eine über das Schwefelatom an den Triazoliumring gebundene Thioethergruppe -SR⁶, eine über das Stickstoffatom an den Triazoliumring gebundene Aminogruppe -NR⁷R⁸₁ eine Acylgruppe - COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R⁴ gemeinsam mit dem Rest R³ eine C₃- bis C₅-Alkylen- oder Alkenylen oder eine C₆- bis C₁₄-Arylen-, eine C₇- bis C₁₄-Aralkylen- oder C₈- bis C₁₄-Aralkenylen-Brücke bildet.

3. Additionsverbindungen von Triazoliumsalzen oder Tetrazoliumsalzen gemäß den Ansprüchen 1 bis 2, in denen X Sauerstoff und R die Methylgruppe ist und in der R¹, R³ und R⁴ die in den Ansprüchen 1 und 2 genannte Bedeutung haben.

4. 1,3,4-Triphenyl-5-H-5-methoxy-1,2,4-triazolin.

5. Thermolyseprodukt der Additionsverbindungen von Triazoliumsalzen oder Tetrazoliumsalzen gemäß den Ansprüchen 1 bis 4, erhältlich durch Erhitzen dieser Additionsverbindungen in Substanz oder in einem inerten, hochsiedenden Lösungsmittel auf Temperaturen von 50 bis 160°C.

6. Verfahren zur Herstellung der Additionsverbindungen von Triazoliumsalzen oder Tetrazoliumsalzen gemäß den Ansprüchen 1 bis 4, dadurch gekennzeichnet, daß man ein Triazoliumsalz der Formel I 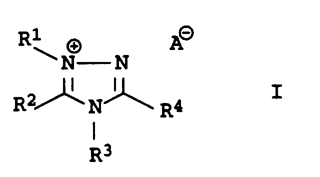 oder ein Tetrazoliumsalz der Formel II 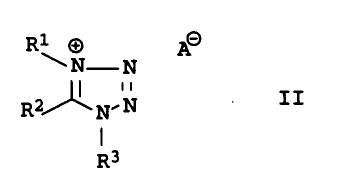 in denen
R¹ und R³ gleich oder verschieden sind und für aliphatische Gruppen mit 1 bis 30 Kohlenstoffatomen, für gegebenenfalls substituierte Arylgruppen, für gegebenenfalls substituierte Aralkylgruppen und/oder für gegebenenfalls substituierte Heteroarylgruppen stehen, und
R² Wasserstoff, die Hydroxymethylengruppe -CH₂OH oder die Hydroxy-hydroxymethylen-methylidin-Gruppe -CH (OH) (CH₂OH) darstellt, und in der
R⁴ Wasserstoff, ein Halogenatom, eine Nitro- oder Cyanogruppe, eine aliphatische Gruppe mit 1 bis 30 Kohlenstoffatomen, eine gegebenenfalls substituierte Arylgruppe, eine gegebenenfalls substituierte Aralkylgruppe, eine gegebenenfalls substituierte Heteroarylgruppe, eine Alkoxygruppe -OR⁵, gegebenenfalls substituierte Aralkylgruppe, eine gegebenenfalls substituierte Heteroarylgruppe, eine Alkoxygruppe -OR⁵, eine Thioethergruppe -SR⁶, eine Aminogruppe -NR⁷R⁸, eine Acylgruppe -COR⁹ oder eine Estergruppe -COOR¹⁰ bedeutet, wobei die Reste R⁵, R⁶, R⁷, R⁸ und R⁹ für Reste stehen, wie sie für R¹ genannt wurden und R¹⁰ eine C₁- bis C₃₀-Alkyl- oder eine gegebenenfalls substituierte Aryl- oder Aralkylgruppe ist,
oder in der der Rest R³ gemeinsam mit dem Rest R⁴ eine C₃- bis C₅-Alkylen- oder Alkenylen- oder eine C₆- bis C₁₄-Arylen-, eine C₇- bis C₁₄-Aralkylen- oder C₈- bis C₁₄-Aralkenylen-Brücke bildet, und
A das Äquivalent eines ein- oder mehrfach negativ geladenen Anions zur elektrischen Neutralisierung der Ladung des Triazoliumkations oder Tetrazoliumkations ist,
in einem Lösungsmittel mit einem Alkanolat oder Thiolat der Formel
RXMe,
in der Me das Grammäquivalent eines Metallkations ist und X und R die in Anspruch 1 genannte Bedeutung haben, bei 0 bis 100°C umsetzt.

## Claims

1. Addition compounds of triazolium salts or tetrazolium salts of the formula III 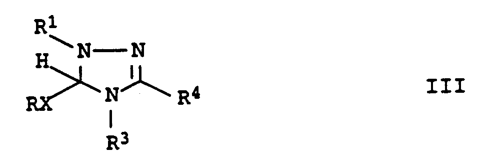 or of the formula IV 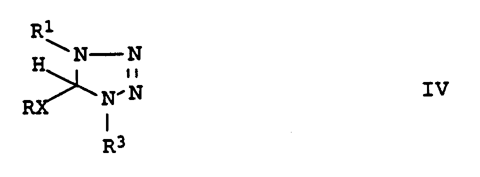
where X is oxygen or sulfur and R is C₁- to C₄-alkyl,
R¹ and R³ are identical or different and represent aliphatic groups having 1 to 30 carbon atoms, optionally substituted aryl groups, optionally substituted aralkyl groups and/or optionally substituted heteroaryl groups, and
R⁴ is hydrogen, a halogen atom, a nitro or cyano group, an aliphatic group having 1 to 30 carbon atoms, an optionally substituted aryl group, an optionally substituted aralkyl group, an optionally substituted heteroaryl group, an alkoxy group -OR⁵, a thioether group -SR⁶, an amino group -NR⁷R⁸, an acyl group -COR⁹ or an ester group -COOR¹⁰, the radicals R⁵, R⁶, R⁷, R⁸ and R⁹ representing radicals as mentioned for R¹ and R¹⁰ being a C₁- to C₃₀-alkyl or an optionally substituted aryl or aralkyl group,
or where the radical R³ joins with the radical R⁴ to form a C₃- to C₅-alkylene or alkenylene or a C₆- to C₁₄-arylene, a C₇- to C₁₄-aralkylene or C₈- to C₁₄-aralkenylene bridge.

2. Addition compounds of triazolium salts or tetrazolium salts as set forth in claim 1, wherein
R¹ and R³ are identical or different and represent C₁- to C₃₀-alkyl groups, C₂- to C₃₀-alkenyl or alkynyl groups having 1 or 2 multiple bonds, C₃- to C₂₀-cycloalkyl or alkenyl groups, C₃- to C₂₀-heterocycloalkyl or -alkenyl groups, C₂- to C₃₀-alkoxy groups having one or more oxygen atoms in the ether chain and being attached to the triazolium ring via a carbon atom, fluorine-, chlorine- and/or bromine-containing C₁-C₃₀-haloalkyl groups having one or more halogen atoms, C₂- to C₃₀-secondary or C₃- to C₃₀-tertiary amino groups attached to the triazolium ring via a carbon atom, optionally substituted C₆- to C₁₄-aryl groups, optionally substituted C₆- to C₁₄-aralkyl groups, optionally substituted C₇- to C₂₀-aralkyl groups, optionally substituted C₂- to C₁₅-heteroaryl groups having 1 to 3 nitrogen atoms or an oxygen or sulfur atom or having 1 to 2 nitrogen atoms and an oxygen or sulfur atom in the ring,
and the radical R⁴ is identical to or different from the radicals R¹ or R³ or denotes a hydrogen atom, a nitro or cyano group, a fluorine, chlorine or bromine atom, an alkoxy group -OR⁵ attached to the triazolium ring via the oxygen atom, a thioether group -SR⁶ attached to the triazolium ring via the sulfur atom, an amino group -NR⁷R⁸₁ attached to the triazolium ring via the nitrogen atom, an acyl group -COR⁹ or an ester group -COOR¹⁰, the radicals R⁵, R⁶, R⁷, R⁸ and R⁹ representing radicals as mentioned for R¹ and R¹⁰ being a C₁- to C₃₀-alkyl or an optionally substituted aryl or aralkyl group,
or wherein the radical R⁴ joins with the radical R³ to form a C₃- to C₅-alkylene or alkenylene or a C₆- to C₁₄-arylene, a C₇- to C₁₄-aralkylene or C₈- to C₁₄-aralkenylene bridge.

3. Addition compounds of triazolium salts or tetrazolium salts as set forth in claims 1 to 2, wherein X is oxygen and R is methyl and R¹, R³ and R⁴ are each as defined in claims 1 and 2.

4. 1,3,4-Triphenyl-5-5H-5-methoxy-1,2,4-triazoline.

5. A thermolysis product of the addition compounds of triazolium salts or tetrazolium salts as set forth in claims 1 to 4, obtainable by heating these addition compounds to temperatures of 50 to 160°C in the absence of a solvent or in an inert, high boiling solvent.

6. A process for preparing the addition compounds of triazolium salts or tetrazolium salts as set forth in claims 1 to 4, characterized in that a triazolium salt of the formula I 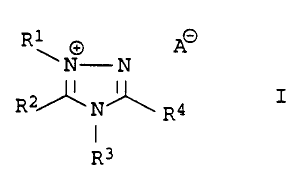 or a tetrazolium salt of the formula II 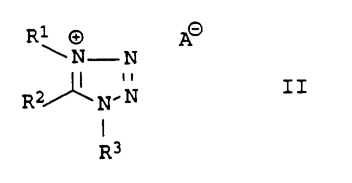 where
R¹ and R³ are identical or different and represent aliphatic groups having 1 to 30 carbon atoms, optionally substituted aryl groups, optionally substituted aralkyl groups and/or optionally substituted heteroaryl groups, and
R² is hydrogen, the hydroxymethylene group -CH₂OH or the hydroxyhydroxymethylenemethylidyne group -CH(OH)(CH₂OH), and where
R⁴ is hydrogen, a halogen atom, a nitro or cyano group, an aliphatic group having 1 to 30 carbon atoms, an optionally substituted aryl group, an optionally substituted aralkyl group, an optionally substituted heteroaryl group, an alkoxy group -OR⁵, a thioether group -SR⁶, an amino group -NR⁷R⁸, an acyl group -COR⁹ or an ester group -COOR¹⁰, the radicals R⁵, R⁶, R⁷, R⁸ and R⁹ representing radicals as mentioned for R¹ and R¹⁰ being a C₁- to C₃₀-alkyl or an optionally substituted aryl or aralkyl group,
or where the radical R³ joins with the radical R⁴ to form a C₃- to C₅-alkylene or alkenylene or a C₆- to C₁₄-arylene, a C₇- to C₁₄-aralkylene or C₈- to C₁₄-aralkenylene bridge, and
A is the equivalent of a singly or multiply negatively charged anion for electrical neutralization of the charge of the triazolium cation or tetrazolium cation,
is reacted with an alkanolate or thiolate of the formula
RXMe,
where Me is the gram equivalent of a metal cation and X and R are each as defined in claim 1, at 0 to 100°C in a solvent.

## Revendications

1. Composés d'addition de sels de triazolium ou de sels de tétrazolium de formule générale III 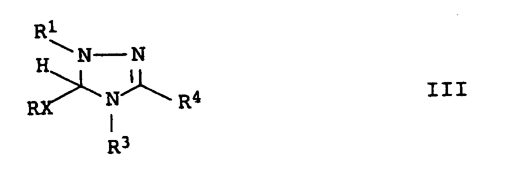 ou de formule IV 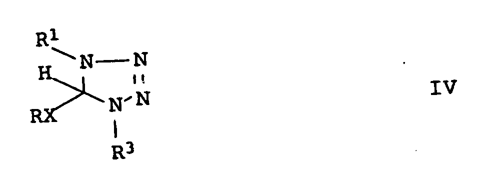
dans lesquelles x est mis pour un atome d'oxygène ou de soufre et R est mis pour un groupement alkyle en C₁-C₄,
R¹ et R³ sont identiques ou différents et sont mis pour des groupements aliphatiques à 1-30 atomes de carbone, pour des groupements aryle éventuellement substitués, pour des groupements aralkyle éventuellement substitués et/ou pour des groupements hétéroaryle éventuellement substitués, et
R⁴ est mis pour un atome d'hydrogène, d'halogène, un groupement nitro ou cyano, un groupement aliphatique à 1-30 atomes de carbone, un groupement aryle éventuellement substitué, un groupement aralkyle éventuellement substitué, un groupement hétéroaryle éventuellement substitué, un groupement alcoxy -OR⁵, un groupement thioéther -SR⁶, un groupement amino -NR⁷R⁸, un groupement acyle -COR⁹ ou un groupement ester -COOR¹⁰, où les restes R⁵, R⁶, R⁷, R⁸ et R⁹ sont mis pour des restes tels que ceux cités pour R¹, et R¹⁰ est un groupement alkyle en C₁-C₃₀ ou un groupement aryle ou aralkyle éventuellement substitués,
ou dans lesquelles R³ forme, avec le reste R⁴, un pont alcénylène ou alkylène en C₃-C₅, ou arylène en C₆-C₁₄, ou aralkylène en C₇-C₁₄, ou aralcénylène en C₈-C₁₄.

2. Composés d'addition de sels de triazolium ou de sels de tétrazolium selon la revendication 1, dans lesquels
R¹ et R³ sont identiques ou différents et sont mis pour des groupements alkyle en C₁-C₃₀, alcényle en C₂-C₃₀ ou alcynyle avec 1 ou 2 liaisons multiples, cycloalcényle ou cycloalkyle en C₃-C₂₀, hétérocycloalcényle ou hétérocycloalkyle en C₃-C₂₀, alcoxy en C₂-C₃₀ avec un ou plusieurs atomes d'oxygène dans la chaîne éther et reliés au cycle triazolium par un atome de carbone, halogénoalkyle en C₁-C₃₀ avec un ou plusieurs atomes d'halogène et contenant du fluor, du chlore et/ou du brome, des groupements amine secondaire en C₂-C₃₀ ou amine tertiaire en C₃-C₃₀ reliés au cycle triazolium par un atome de carbone, des groupements aryle en C₆-C₁₄ éventuellement substitués, des groupements aralkyle en C₆-C₁₄ éventuellement substitués, des groupements aralkyle en C₇-C₂₀ éventuellement substitués, des groupements hétéroaryle en C₂-C₁₅ éventuellement substitués et comportant, dans le cycle, 1 à 3 atomes d'azote, ou un atome d'oxygène ou un atome de soufre ou comportant 1 à 2 atomes d'azote et un atome d'oxygène ou de soufre,
et le reste R⁴ est identique ou différent des restes R¹ ou R³ ou représente un atome d'hydrogène, un groupement nitro ou cyano, un atome de fluor, de chlore ou de brome, un groupement alcoxy -OR⁵ relié au cycle triazolium par l'atome d'oxygène, un groupement thioéther -SR⁶ relié au cycle triazolium par l'atome de soufre, un groupement amino -NR⁷R⁸ relié au cycle triazolium par l'atome d'azote, un groupement acyle -COR⁹ ou un groupement ester -COOR¹⁰, où les restes R⁵, R⁶, R⁷, R⁸ et R⁹ sont mis pour des restes tels que ceux cités pour R¹, et R¹⁰ est un groupement alkyle en C₁-C₃₀, ou un groupement aryle ou aralkyle éventuellement substitués,
ou dans lesquelles R⁴ forme, avec le reste R³, un pont alcénylène ou alkylène en C₃-C₅, ou arylène en C₆-C₁₄, ou aralkylène en C₇-C₁₄, ou aralcénylène en C₈-C₁₄.

3. Composés d'addition de sels de triazolium ou de sels de tétrazolium selon les revendications 1 à 2, dans lesquels X est un atome d'oxygène et R un groupement méthyle, R¹, R³ et R⁴ prenant la signification mentionnée dans les revendications 1 et 2.

4. 1,3,4-triphényl-5-H-5-méthoxy-1,2,4-triazoline.

5. Produit de la thermolyse des composés d'addition de sels de triazolium ou de sels de tétrazolium selon l'une quelconque des revendications 1 à 4, pouvant être obtenus par chauffage de ces composés d'addition dans la masse ou dans un solvant inerte à haut point d'ébullition, à des températures de 50-160°C.

6. Procédé de préparation de composés d'addition de sels de triazolium ou de sels de tétrazolium selon l'une quelconque des revendications 1 à 4, caractérisé en ce que l'on fait réagir un sel de triazolium de formule I 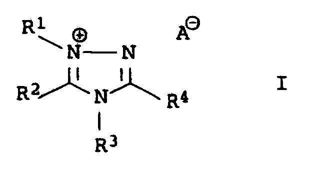 ou un sel de tétrazolium de formule II 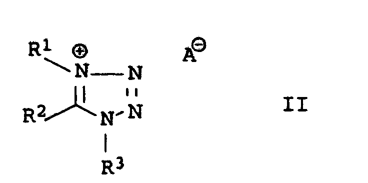 dans lesquelles
R¹ et R³ sont identiques ou différents et sont mis pour des groupements aliphatiques à 1-30 atomes de carbone, pour des groupements aryle éventuellement substitués, pour des groupements aralkyle éventuellement substitués et/ou pour des groupements hétéroaryle éventuellement substitués, et
R² représente un atome d'hydrogène, le groupement hydroxyméthylène -CH₂OH ou le groupement hydroxy-hydroxyméthylène-méthylidine -CH(OH)(CH₂OH) et où
R⁴ est mis pour un atome d'hydrogène, d'halogène, un groupement nitro ou cyano, un groupement aliphatique à 1-30 atomes de carbone, un groupement aryle éventuellement substitué, un groupement aralkyle éventuellement substitué, un groupement hétéroaryle éventuellement substitué, un groupement alcoxy -OR⁵, un groupement thioéther -SR⁶, un groupement amino -NR⁷R⁸, un groupement acyle -COR⁹ ou un groupement ester -COOR¹⁰, où les restes R⁵, R⁶, R⁷, R⁸ et R⁹ sont mis pour des restes tels que ceux cités pour R¹, et R¹⁰ est un groupement alkyle en C₁-C₃₀, ou un groupement aryle ou aralkyle éventuellement substitués,
ou dans lesquelles R³ forme, avec le reste R⁴, un pont alcénylène ou alkylène en C₃-C₅, ou arylène en C₆-C₁₄, ou aralkylène en C₇-C₁₄, ou aralcénylène en C₈-C₁₄ et
A est l'équivalent d'un anion avec une ou plusieurs charges négatives, afin de compenser la charge électrique du cation triazolium ou tétrazolium,
dans un solvant avec un alcanolate ou un thiolate de formule
RXMe
dans laquelle Me est l'équivalent-gramme d'un cation métallique et X et R ont la signification mentionnée dans la revendication 1, à 0-100°C.
